# EUROPEAN PATENT SPECIFICATION

(11) **EP 3 939 633 B1**
(45) Date of publication and mention of the grant of the patent: **04.11.2026**
(21) Application number: 21190542.7
(22) Date of filing: 20.09.2017
(51) Int. Cl.: A61M 1/02, A61M 1/36

(54) **DISPOSABLE FLUID CIRCUITS FOR EXTRACORPOREAL PHOTOPHERESIS WITH INTEGRATED SOURCE OF PHOTOACTIVE AGENT**
EINWEG-FLÜSSIGKEITSKREISLÄUFE FÜR DIE EXTRAKORPORALE PHOTOPHERESE MIT INTEGRIERTER QUELLE EINES PHOTOAKTIVEN MITTELS
CIRCUITS DE FLUIDES JETABLES POUR PHOTOPHÉRÈSE EXTRACORPORELLE AYANT UNE SOURCE INTÉGRÉE D'AGENT PHOTOACTIF

(30) Priority: 21.09.2016 US 201662397744 P
(43) Date of publication of application: 19.01.2022
(62) Document number(s) of the earlier application(s) in accordance with Art. 76 EPC: 17192023.4 / 3 300 748
(73) Proprietor: Fenwal, Inc., Lake Zurich, IL 60047 (US)
(72) Inventor: ABEDIN, Tanima Jahan, Lake Zurich, IL 60047 (US); RADWANSKI, Katherine, Lake Zurich, IL 60047 (US)
(74) Representative: Maikowski & Ninnemann Patentanwälte Partnerschaft mbB

(56) References cited:
- EP-A1- 2 620 171
- EP-A1- 2 777 754
- WO-A1-01/68031
- WO-A1-03/049634

## Description

### Field of the Disclosure

The present disclosure is directed to the processing of blood components in the course of an extracorporeal photopheresis treatment. More specifically, the present disclosure is directed to a disposable fluid circuit and its use in extracorporeal photopheresis wherein the circuit includes an integrated source of the photoactive agent, allowing for combining a pre-determined amount of the agent and the collected component with a reduced risk of operator error during such combining.

### Background

Whole blood can be separated into its constituent components (cellular or liquid), and the desired component can be separated so that it can be administered to a patient in need of that particular component. For example, mononuclear cells (MNCs), primarily lymphocytes and monocytes, can be removed from the whole blood of a patient, collected, and subjected to photodynamic therapy in a procedure commonly referred to as extracorporeal photopheresis, or ECP. In ECP, MNCs are treated with a photoactivate or photosensitizing agent (e.g., 8-methoxypsoralen (8-MOP)) and subsequently irradiated with specified wavelengths of light to achieve a desired effect, and returned to the patient for the treatment of various blood diseases to, e.g., eliminate immunogenicity in cells, inactivate or kill selected cells, inactivate viruses or bacteria, or activate desirable immune responses.

In one example of an ECP procedure, blood is withdrawn from the patient and the mononuclear cells are separated (typically by centrifugation) from the remainder of the other whole blood components. The separated mononuclear cells are combined with a selected dose of 8-MOP and subjected to light (typically UV-A) to activate the 8-MOP molecules. The light crosslinks 8-MOP to DNA strands inside the cell and on the cell wall of the exposed mononuclear cells, eventually causing cell apoptosis. The fluid with the altered mononuclear cells is reinfused back into the patient to induce an immune system response. The procedure is carried out using a disposable fluid circuit, i.e., a "kit," that includes devices for accessing the vascular system of the patient (e.g., venipuncture needles), tubing that defines flow paths for conveying fluid to and from the patient to a separation chamber and a treatment container, and solution or storage containers. Examples of a photopheresis methods and systems of the type described above are set forth in U.S. Patent No. 9,399,093 and U.S. Patent Application Publication No. US2014/0370491.

In order to achieve the desired apoptotic response in the MNC, the correct dosage of 8-MOP must be mixed with the collected MNCs. Current ECP systems are designed with target 8-MOP concentrations of approximately 100-400 ng/mL in the collected (and diluted) cells, which can be achieved using existing 8-MOP formulations provided at a concentration of 20 µg/mL in 10 mL glass vials. If a lower 8-MOP dosage were to be injected, the therapy may be less effective. On the other hand, if a higher 8-MOP dosage is administered, certain patient side effects related to 8-MOP, such as irritation to the skin and eyes due to exposure to sunlight (and the further potential for erythema, cataracts, or skin carcinoma resulting from such exposure to UVA light) may occur and may be severe.

In current ECP systems, the operator uses a syringe to inject the photoactive agent (e.g., 8-MOP) into the container that holds the collected MNCs. Thus, the responsibility to prepare and correctly inject the 8-MOP with the correct concentration and volume lies with the operator. The operator may likewise be responsible for the correct storage and correct aseptic techniques when drawing and injecting, as well as the proper disposal of the drug. If the wrong agent were added or too much of the agent were added, there may be risk to the patient, as described above. In the event of the wrong agent being added, the consequences of such administration could be significant and even catastrophic.

Accordingly, if the treating agent were already incorporated into the disposable fluid circuit, many of these risks can be mitigated and the tasks performed by the operator would also be facilitated. Incorporating a pre-determined dose of the photoactive agent and (preferably) allowing for automated opening of the flow paths between the container that holds the 8-MOP and a treatment container would ensure that the correct dosage of this drug is used and reduces the risk of bacterial contamination, particularly in already immune-suppressed patients (e.g. GVHD patients). EP2620171 A1 discloses systems and methods for performing extracorporeal photopheresis. WO03/049634 A1 and WO01/68031 A1 disclose further systems for blood treatment. EP2777754 discloses an automated frangible cannula breaker.

### Summary

The invention is defined in independent claims 1 and 6. Preferred embodiments are specified in the dependent claims. There are several aspects of the subject matter described herein. In one aspect, the subject matter of this disclosure is directed to a disposable fluid circuit for combined association with a cell separator and a separate irradiation device. The fluid circuit includes at least one patient access device for establishing flow communication with a patient, a separation chamber in openable fluid communication with the patient access device and a treatment container including walls made of a material that is transparent to light of a selected wavelength. Tubing defining a sterile flow path between the separation chamber and the treatment container is provided and includes a length sufficient to allow for placement of the separation chamber within the separator, and placement of the treatment container in the separate irradiation device. A container of photoactive agent is integral with the fluid circuit, the container having an outlet in openable flow communication with an outlet flow path that includes at least one frangible cannula for preventing flow of photoactive agent from the container of photoactive agent to the treatment container.

In another aspect, the subject matter of this disclosure is directed to a system for performing a therapeutic cellular treatment wherein the system includes a reusable separator unit for separating blood into blood components, a reusable irradiation unit for treating a separated blood component, a controller configured to effect the separation of blood into blood components, effect the conveying of the separated blood components through a disposable fluid circuit and, optionally, effect the treatment of a separated component. The system includes a disposable fluid circuit for combined association with the separator and the separate irradiation unit.

The fluid circuit includes one or more patient access devices for establishing flow communication with a patient, a separation chamber in openable fluid communication with the one or more patient access devices, a treatment container made of walls that are transparent to light of a selected wavelength and tubing defining a sterile flow path between the separation chamber and the treatment container. The flow path preferably has a length sufficient to allow for placement of the separation chamber within the separator and allow for placement of said treatment container in the separate irradiation device. A container of photoactive agent integral with the fluid circuit has an outlet in openable flow communication with an outlet flow path. A frangible cannula for preventing flow of photoactive agent from the treating agent container with a photoactive agent to said treatment container is included at the outlet or in the outlet flow path. The system may further include an automated cannula breaker configured to receive the cannula.

In a further aspect, the subject matter described herein is directed to a method for combining a treating agent with a collected blood component. None of the methods described herein form part of the invention. The method includes mounting an integral disposable fluid circuit including one or more vascular access devices, a separation chamber, a treatment container and a container of a treating agent on a separator unit and an irradiation unit wherein the separation chamber is mounted within the separator unit and the treatment container is mounted within the irradiation unit. The method includes introducing whole blood into the separation chamber and separating whole blood into blood components wherein one of the blood components comprises mononuclear cells. The mononuclear cells are removed from the separation chamber and conveyed from the separation chamber to the treatment container. A flow path between the treating agent container and the treatment container is opened to combine the treating agent with the mononuclear cell product.

### Brief Description of the Drawings

Figure 1 is a diagram of the system and method for processing a mononuclear cell product in accordance with the present disclosure;
Figure 2 is a partial perspective view of the front panel of a multifunctional apheresis separator useful in the methods and systems described herein with a portion of the disposable fluid circuit mounted thereon;
Figure 3 is a diagram of one embodiment of a disposable fluid circuit suitable for use with the system described herein;

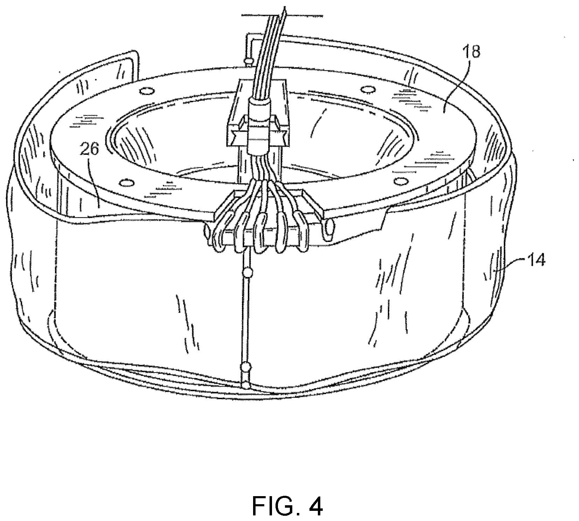

Figure 4 is a perspective view of a container with separation chamber of the fluid circuit used with a separator;
Figure 5 is a schematic view of the control circuitry, including the controller of the system of Fig. 1;
Figures 6(a)-6(b) are enlarged views of a treatment container and treating agent container of the fluid circuit of Fig. 3;
Figure 7 is a diagram of another embodiment of a disposable fluid circuit suitable for use with the system of the present disclosure;
Figure 8 is an enlarged view of a treatment container of the fluid circuit of Fig. 7 with the integral, in-line, treating agent container in openable flow communication therewith;
Figure 9 is a diagram of still another embodiment of a disposable fluid circuit suitable for use with the system of the present disclosure;
Figure 10 is a perspective view of an automated cannula breaker for use with the system of the present disclosure;
Figure 11 is a view of the automated cannula breaker in association with an in-line pouch or bag of photoactive agent;
Figure 12 is a flow chart setting forth the method of combining a treating agent with a collected blood component using, for example, the disposable fluid circuit of Fig. 3; and
Figure 13 is a flow chart setting forth the method of combining a treating agent with a collected blood component using, for example, the disposable fluid circuit of Figs. 7 and 8.

### Detailed Description of the Embodiments

The present disclosure is directed to disposable fluid circuits, methods, and systems for the processing of mononuclear cells in an ECP treatment. The circuits, methods, and systems of the present disclosure are described in connection with particular apheresis and irradiation/illumination devices for purposes of exemplification only. It will be understood that the methods and systems described and claimed herein may be carried out and provided in combination with other apheresis and/or irradiation/illumination devices that will be known to those of skill in the art.

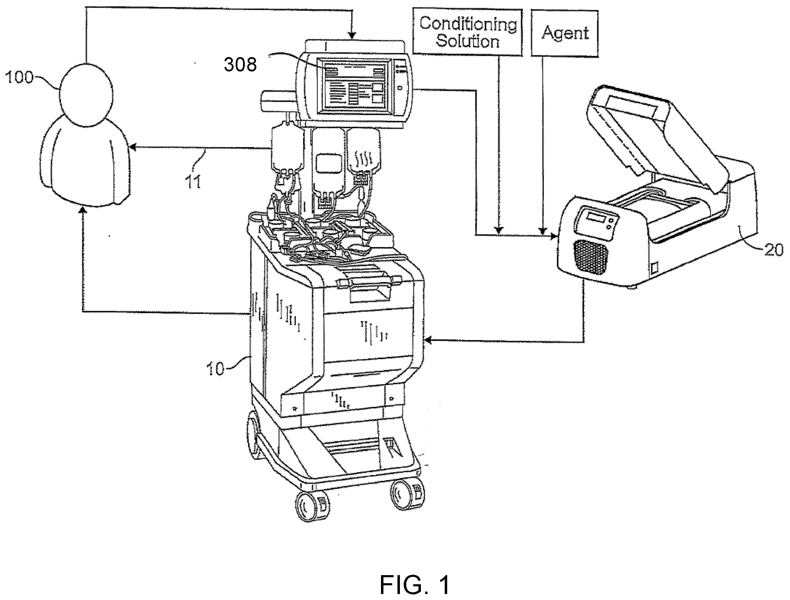

Turning now to the Figures, Fig. 1 diagrammatically shows a system and the flow of fluid in the method described herein. In accordance with the present disclosure, the system includes a reusable separation unit or separator 10 and a treatment or irradiation unit 20. In one embodiment, irradiation unit 20 is independent and housed separately from separator 10. Although separately housed and shown as independent, stand-alone devices, it is preferable that separator 10 and irradiation device 20 be located adjacent to each other. While Fig. 1 shows a preferred embodiment of the individual separation and irradiation units, it will be appreciated that the methods described herein may also be used with devices having integrated separation and irradiation components housed in one device.

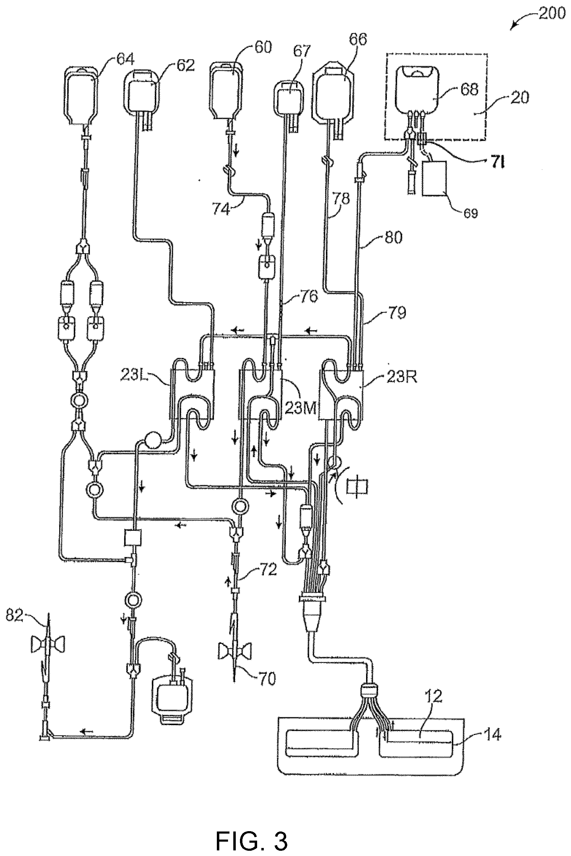

As generally shown in Fig. 1, whole blood is withdrawn from the patient 100 and introduced into the separator 10 where the whole blood is separated to provide a target cell population. More particularly, whole blood is withdrawn from the patient through venipuncture needle 82 (Fig. 3) and introduced into the separation chamber of separation container 12 carried within and/or mounted on a centrifuge device of separator 10. Within the separator 10, the target cell population is separated from other components. In a preferred embodiment in accordance with the present disclosure, the target cell population is the patient's mononuclear cells (MNC). Other components separated from the whole blood in this initial separation, such as red blood cells, plasma and platelets, may be returned to the patient or collected in pre-attached containers of the blood processing set, as shown by line 11. The collection of mononuclear cells is more specifically described in U.S. Patent No. 6,027,657.

The separated target cell population, e.g., mononuclear cells with residual red blood cells and plasma, is then prepared for treatment and irradiation in reusable treatment component or irradiation unit 20. In accordance with the present disclosure, effective treatment of the mononuclear cells with ultraviolet light requires that the collected mononuclear cells be provided in a suspension having a suitable hematocrit, i.e., a certain (low) concentration of red blood cells. Specifically, the hematocrit level in the MNC suspension to be treated affects the amount of UV light that the MNC are exposed to as the red blood cells in the MNC suspension will block at least a portion the UV light from reaching the targeted MNCs. The hematocrit level of the MNC product to be treated may be adjusted by diluting the collected MNC product with plasma and/or saline, as described in U.S. Patent No. 9,399,093 and U.S. Patent Application Publication No. US2014/0370491.

Turning now, more specifically, to one embodiment of the reusable hardware units and disposable fluid circuit components of the system, devices/separators 10 useful in the collection (and washing) of mononuclear cells include the Amicus^{®} Separator made and sold by Fresenius-Kabi USA, of Lake Zurich, Illinois. Mononuclear cell collections using a device such as the Amicus^{®} are described in greater detail in U.S. Patent No. 6,027,657.

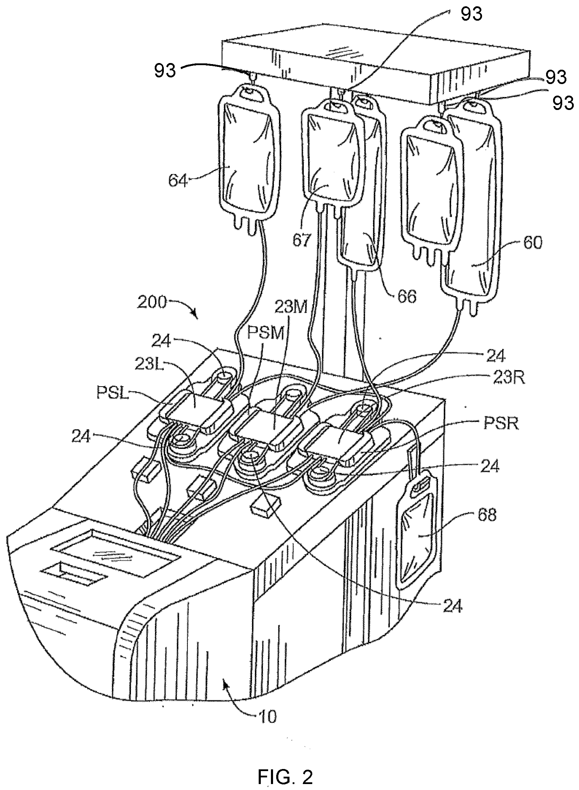

Briefly, Fig. 2 shows a representative blood centrifuge device/ separator 10 with fluid circuit 200 mounted thereon. The fluid circuit 200 includes a blood processing container 14 (see Fig. 3) defining a separation chamber 12 suitable for harvesting mononuclear cells from whole blood. As shown in Fig. 2, a portion of disposable processing set or fluid circuit 200 is mounted on the front panel of device/ separator 10. As also shown in Fig. 3, separation chamber 12, which is integral with the rest of circuit 200, is defined by the walls of a flexible processing container 14 carried within an annular gap defined by a rotating spool element 18 and an outer bowl element (not shown) housed within the cabinet of device/separator 10. The processing container 14 takes the form of an elongated tube or belt which is wrapped about the spool element 18 before use. The bowl and spool element 18 are pivoted on a yoke between an upright position and a suspended position, also not shown. In operation, the centrifuge device within separator 10 rotates the suspended bowl and spool element 18 about an axis, creating a centrifugal field within the processing chamber of container 14. Details of the mechanism for causing relative movement of the spool 18 and bowl elements as just described are disclosed in U.S. Patent No. 5,360,542 entitled "Centrifuge with Separable Bowl and Spool Elements Providing Access to the Separation Chamber."

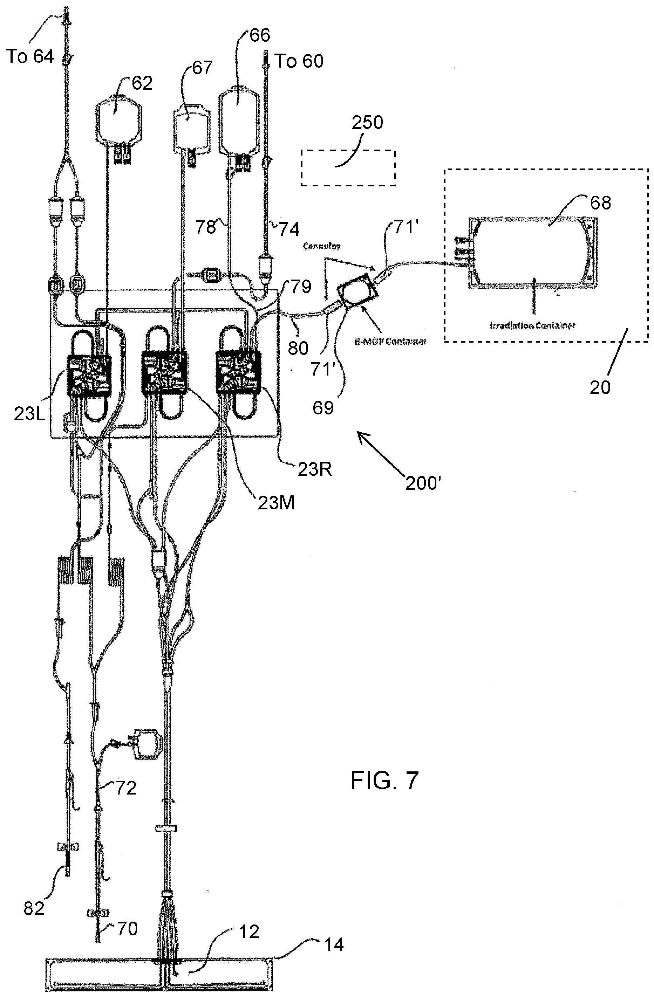

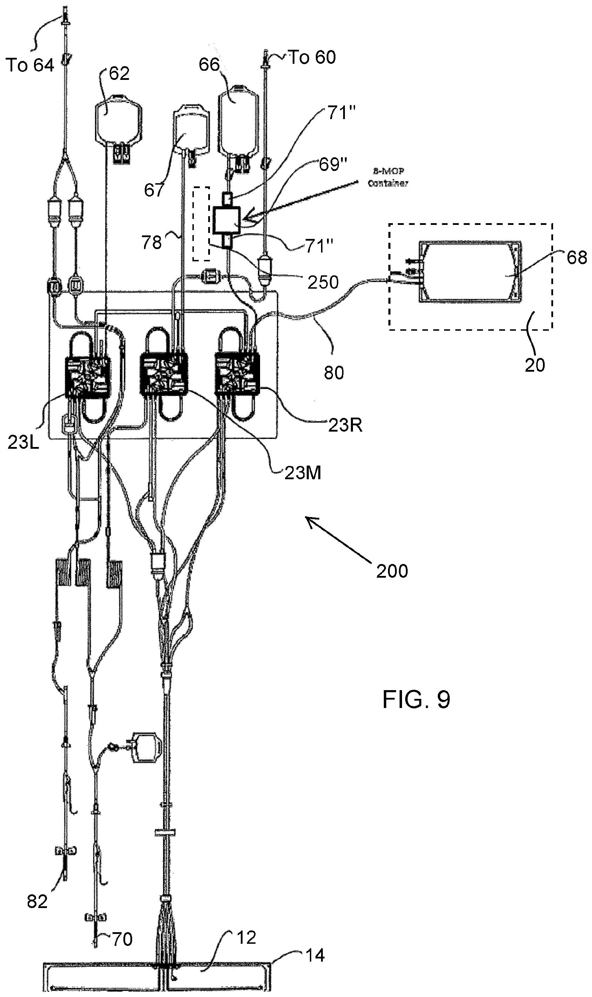

With reference to Figs. 2-3 (as well as Figs. 7 and 9), fluid circuit 200 (200', 200") includes a plurality of processing fluid flow cassettes 23L, 23M and 23R with tubing loops for association with peristaltic pumps 24 on device 10. As described in U.S. Patent No. 6,027,657, cassettes 23L, 23M and 23R include molded plastic bodies with integrally molded liquid flow channels. Valve stations (depicted as numbered circles within each cassette and best seen in Fig. 7) are molded into the backside of cassette bodies. A flexible diaphragm covers and seals the backside of the cassette (23) body. Valve stations align with valve actuators of pump stations (PSL, PSM and PSR) located on the front panel of device 10. Fluid circuit 200 also includes a network of tubing and pre-connected containers for establishing flow communication with the patient and for processing and collecting fluids and blood and blood components, as shown in greater detail in Fig. 2.

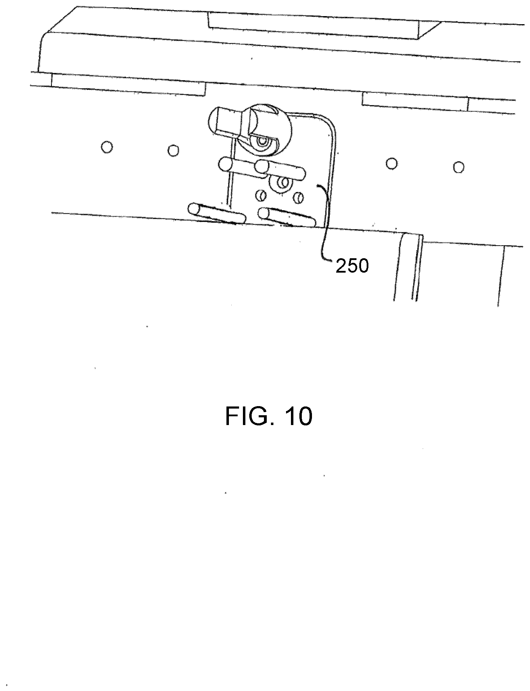

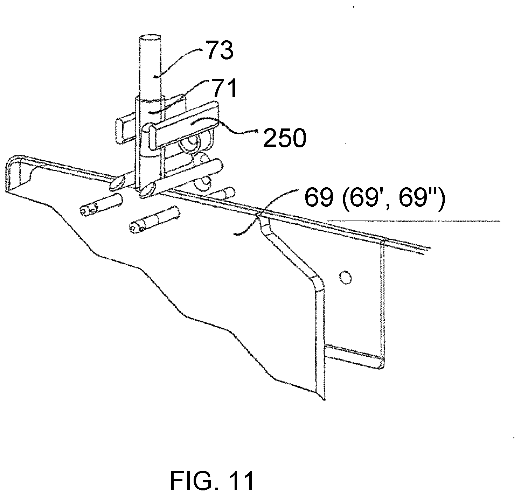

The system described herein may, optionally, further include one or more automated cannula breaker(s) 250, as shown in Figs. 10-11 and described in U.S. Patent Application Publication No. US2014/0263529. Cannula breaker is configured to receive frangible cannulas commonly used in blood processing fluid circuits to open otherwise closed fluid paths. In accordance with the system described herein, cannula breakers 250 may be a stand-alone unit or more preferably, may be integral with or otherwise carried by separation unit 10 or irradiation unit 20. As described below, automated cannula breakers may relieve the operator of manually breaking the frangible cannulas such as those located between the container 69 of treating agent and treatment container 68.

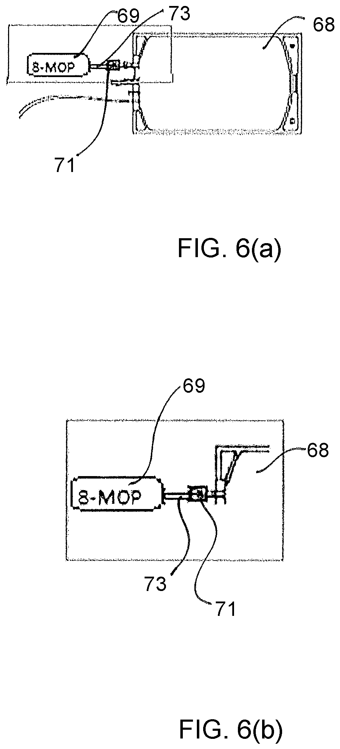

As further seen in Figs. 2 and 3, in one embodiment disposable processing set 200 may include a container 60 for supplying anticoagulant, a waste container 62 for collecting waste from one or more steps in the process for collecting, treating and/or washing mononuclear cells, a container 64 for holding saline or other priming or conditioning medium, a container 66 for collecting plasma, a container 68 for collecting the mononuclear cells and, preferably, container or pouch 69 for holding the photoactivation agent. As shown in Fig. 3 and, in greater detail, in Figs. 6(a) and 6(b), container 69 may be integrally pre-attached to treatment container 68 and in openable fluid communication therewith. A frangible cannula 71 may be included in flow path 73 between containers 68 and 69 and broken by the operator to combine the photoactive agent with the collected MNCs in container 68. Alternatively, the portion of the flow path between containers 68 and 69 may be mounted onto automated cannula breaker 250 described above and shown in Figs. 10-11, and described in greater detail in U.S. Patent Application Publication No. US2014/0263529. Cannula breaker 250 may be activated to open flow path 73 by operator control or by a pre-programmed command received from the system controller (described below) at a pre-determined time. For example, once weight scale 93 holding container 68 detects that a suitable volume of MNCs has been collected, controller 300 may effect breakage of cannula 71, thereby establishing flow from container 69 to container 68.

Container 68 may also serve as the illumination container, and is preferably pre-attached to the disposable circuit 200. Alternatively, container 68 may be attached to set 200 by known sterile connection techniques, such as sterile docking or the like. With reference to Fig. 3, fluid circuit includes inlet line 72, an anticoagulant (AC) line 74 for delivering AC from container 60, an RBC line 76 for conveying red blood cells from chamber 12 of container 14 to container 67, a platelet-poor plasma (PPP) line 78 for conveying PPP to container 66 and line 80 for conveying mononuclear cells to and from separation chamber 14 and collection/illumination container 68. The fluid processing circuit includes one or more patient access device(s) such as venipuncture needle(s) for accessing the circulatory system of the patient. As shown in Fig. 3, fluid circuit 200 includes inlet needle 70 and return needle 82. In an alternative embodiment, a single needle can serve as both the inlet and outlet needle.

Container 68 is suitable for irradiation by light of a selected wavelength. By "suitable for irradiation" it is meant that the walls of the container are sufficiently transparent to light of the selected wavelength to activate the photoactive agent. In treatments using UVA light, for example, container walls made of ethylene vinyl acetate (EVA) are suitable. Accordingly, as indicated above, container 68 in which the mononuclear cells are collected may serve both as the collection container and the irradiation container. Container 68 may be placed inside irradiation unit 20 by the operator or, more preferably, may be placed inside the irradiation chamber of irradiation device 20 at the beginning of the ECP procedure and prior to whole blood withdrawal (as shown by the broken lines representing device 20 in Fig. 2). In any event, container 68 preferably remains integrally connected to the remainder of fluid circuit 200 during the entire procedure, thereby maintaining the closed or functionally closed condition of fluid circuit 200.

Fluid flow through fluid circuit 200 is preferably driven, controlled and adjusted by a microprocessor-based controller in cooperation with the valves, pumps, weight scales and sensors of device 10, the details of which are described in the previously mentioned U.S. Patent No. 6,027,657. As described below, the controller is programmed to activate rotation of pumps (and control the rotational speed thereof), associated with cassettes 23L, 23M and 23R, open and close valves, receive output signals from sensors and detectors, such as the interface detection system described below, and preferably, to commence and control treatment of the MNC in irradiation unit 20.

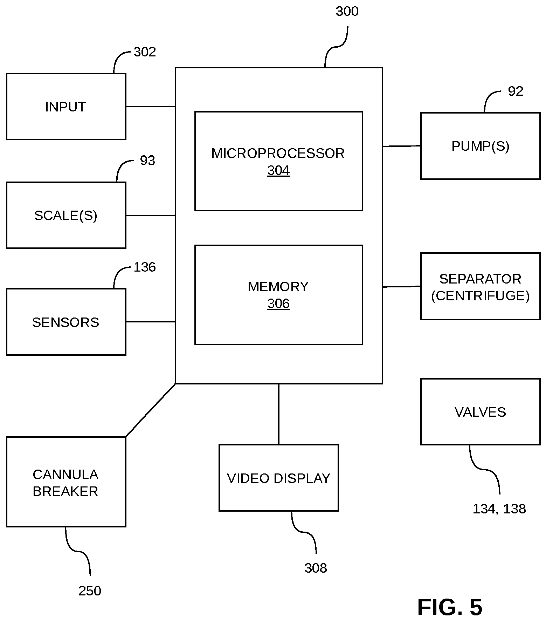

Fig. 5 is a schematic view of the control unit or "controller" 300 included in device 10 of the present disclosure. The controller 300 may include a microprocessor 304 (which may include multiple physical and/or virtual processors). According to other embodiments, the controller 300 may include one or more electrical circuits designed to carry out the actions described herein. In an embodiment, controller 300 may include a microprocessor and other circuits or circuitry. In addition, the controller 300 may include one or more memories 306. The instructions by which the microprocessor 304 is programmed may be stored on the memory 306 associated with the microprocessor 304, which memory/memories 306 may include one or more tangible non-transitory computer readable memories, having computer executable instructions stored thereon, which when executed by the microprocessor 304, may cause the microprocessors 304 to carry out one or more actions as described herein.

As is also illustrated in Fig. 5, controller 300 may be coupled to one or more of the structures described herein, for example to receive information (e.g., in the form of signals) from these structures or to provide commands (e.g., in the form of signals) to these structures to control the operation of the structures. As illustrated in Fig. 5, the controller 300 may be coupled to weight scales 93 (seen in Fig. 3) that hold solution containers or that are provided to collect separated blood components, the sensors associated with device 10, or more specifically with the cassettes 24L, 24M, and 24R, the valve assemblies 132, and the at least one input 302 to receive information from those devices. Additionally, the controller 300 may be coupled to the pumps 24 and the separator (centrifuge) drive unit (not shown) to provide commands to those devices and to control their operation. As further shown in Fig. 5, controller 300 may also be coupled to automated cannula breaker 250. It may also be possible that the controller 300 receives information from and provides commands to a given structure, such as one of the structures already mentioned. The controller 300 may be directly electrically connected to these structures to be coupled to them, or the controller 300 may be directly connected to other intermediate equipment that is directly connected to these structures to be coupled to them.

The at least one input 302 may include a number of different devices according to the embodiments described herein. For example, the input 302 could include a keyboard or keypad by which a user may provide information and/or instructions to the controller 300. Alternatively, the input 302 may be a touch screen, such as may be used in conjunction with a video display 308 (Fig. 1) that is disposed on the front panel of the device 10, the video display 308 also being coupled to the controller 300. The assembly of the input/touch screen 302 and video display 308 may be one of the aforementioned structures to which the controller 300 is coupled from which the controller 300 receives information and to which the controller 300 provides commands.

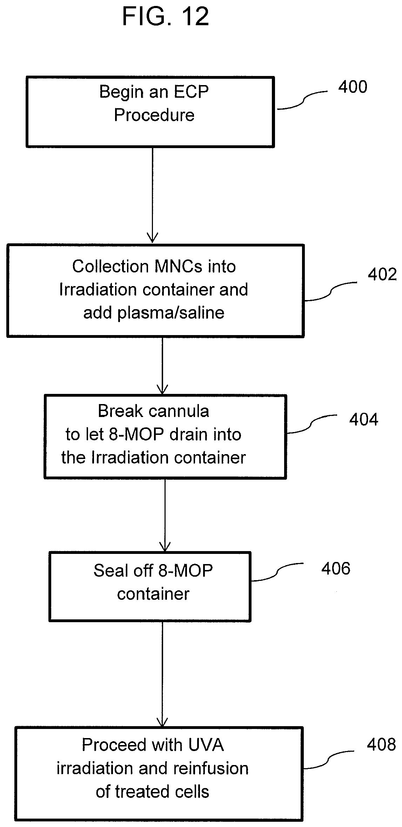

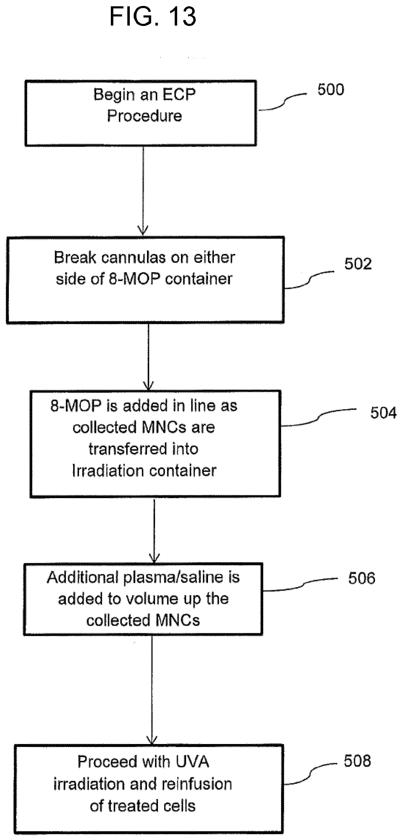

The method of using the Fig. 3, 6(a) and 6(b) embodiment of disposable fluid circuit 200 to combine the photoactive agent with the collected MNCs in container 68 is generally set forth in Fig. 13. As shown in Fig. 12, upon commencement of an ECP procedure (step 400), MNCs are collected in in treatment container 68 and additional plasma and/or saline may added, as necessary, to arrive at a treatment-ready MNC product (step 402). Cannula 71 is then broken to allow the photoactive agent within container 69 to drain into container 68 (step 404). As described above, breaking of frangible cannula 71 may be performed manually by the operator or automatically by an automated cannula breaker 250, either by an operator-entered instruction or under the command of controller 300 once the system has sensed that a predetermined amount of MNCs has been collected in treatment container 68. The amount or concentration of photoactive agent in container 69 may be pre-determined based on the volume of collected MNCs and conditioning fluid. While the volume of the treating agent is fixed, the number of collected MNCs per procedure may vary. The system may be programmed to receive targeted collection amounts of MNCs and may further be programmed to cease the collection cycle once the target has been reached. Once the treating agent has drained into treatment container 68, container 69 may be sealed off from the remainder of the circuit (Step 406) and treatment of the collected cell product may proceed (Step 408).

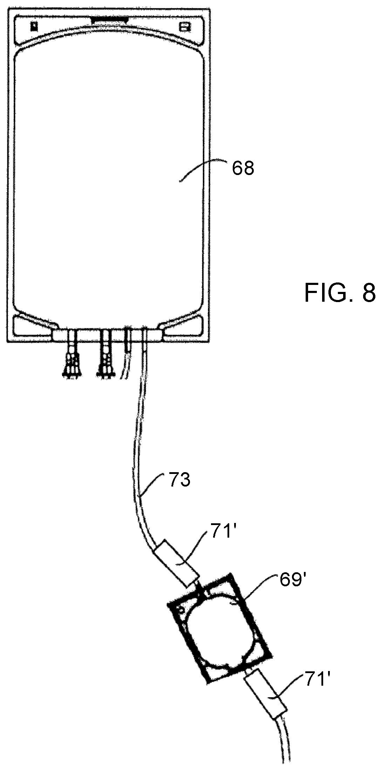

Figs. 7 and 8 show a different embodiment of a disposable fluid circuit 200' that can include an integrated treating agent container 69'. In Figs. 7 and 8, container 69' is shown in-line with the tubing 80 that defines a flow path and provides flow communication between separation chamber 12 of container 14 and treatment container 68. Fluid circuit 200' includes a frangible cannula located at the outlet of container 69' or located elsewhere between treating agent container 69' and treatment container 68. Fluid circuit 200' may include a second frangible cannula 71' located at the inlet of treatment container 68 or located elsewhere between treatment container 68 and treating agent container 69'.

The method of using disposable fluid circuit 200' is generally set forth in Fig. 13. Upon commencement of the ECP procedure (step 500), both cannulas 71' are broken at or just before the time that the separated MNCs from the first cycle of normal MNC collection begin exiting (pumped from) separation chamber of separation container 14. As MNCs from separation chamber 12, through cassette 24R and now-open line 80, they enter in-line treating agent container 69' where they are mixed with the photoactive agent and the mixture is collected in treating container 68 (step 504). As in the method of using disposable fluid circuit of Figs. 3 and 6(a) and 6(b), the concentration of photoactive agent in container 69 may be pre-determined based on the volume of collected MNCs to which plasma and/or saline are or have been added to arrive at a treatment-ready MNC product having a suitable hematocrit for effective treatment.

As described in connection with the earlier embodiments, breaking of frangible cannula 71' may be manually performed by the operator or automatically by an automated cannula breaker 250, either by an operator-entered instruction or under the command of controller 300 once the system has sensed that MNCs are being removed from separation chamber 12. For example, where an automated cannula breaker is used, breakage may be prompted by the optical sensor sensing the MNCs traveling through a predetermined portion of the disposable fluid circuit flow path.

A further alternative embodiment of a disposable fluid circuit 200" with an integrated treating agent container 69" is shown in Fig. 9. As in the embodiment of Figs. 7 and 8, treating container 69" is an in-line container integral with fluid circuit 200". However, in this embodiment, container 69" is located between plasma container 66 and cassette 23R. Plasma container collects plasma separated from whole blood during initial mononuclear cell collection. The plasma collected in container 66 may be used to adjust the volume of the collected MNCs and arrive at a suitable hematocrit. Saline from the initial priming of circuit 200" may likewise be collected in plasma container 66 and used to adjust the volume of the MNC product and arrive at a treatment-ready MNC product, as described in simultaneously filed provisional patent application entitled "METHODS AND SYSTEMS FOR MAINTAINING PATIENT FLUID BALANCE DURING AN EXTRACORPOREAL THERAPEUTIC CELL TREATMENT, identified by Attorney Docket number F-6782 (9360-0547), published in the non-provisional application US2018/0078696. Similar to the embodiment of Figs. 7 and 8, treating agent container 69" includes two frangible cannulas 71" located on the either side of the container either at the inlet and outlet elsewhere between plasma container 66 and treating agent container 69" and between treating agent container 69" and cassette 23R. As described in connection with the earlier embodiments, breaking of frangible cannulas 71" may be manually performed by the operator or automatically by an automated cannula breaker 250, either by operator instruction or under the command of controller 300, once the system has sensed that platelet poor plasma is being removed from separation chamber 12 during initial whole blood processing or when saline that was used to prime circuit 200" is being diverted to plasma container 66. Thus, in this embodiment, the treating agent is combined with the diluting solution (e.g., plasma/saline) and subsequently conveyed to treatment container 68 and the amount of the treating agent delivered to the MNC can be varied by pumping more or less of the diluting solution to the MNC in treatment container 68 (whereas in the previous embodiments, the amount of treating agent delivered is fixed).

## Claims

1. A disposable fluid circuit (200', 200") for combined association with a cell separator and a separate irradiation device comprising:
a) a patient access device for establishing flow communication with a patient;
b) a cell separation chamber (12) in openable fluid communication with said patient access device;
c) a treatment container (68) comprising walls made of a material that is transparent to light of a selected wavelength;
d) a processing fluid flow cassette (23R) including a molded plastic body with integrally molded liquid flow channels and valve stations molded into the backside of the cassette body;
e) further comprising a container (66) including plasma and/or saline, for adjusting the volume of a collected component in said treatment container (68) in openable flow communication with said processing fluid flow cassette (23R) and said treatment container (68),
f) tubing defining a sterile flow path between said separation chamber, said processing cassette (23R) and said treatment container (68), said flow path having a length sufficient to allow for placement of said separation chamber within said separator and allow for placement of said treatment container in said separate irradiation device;
g) a container of photoactive agent (69) integral with said circuit, said container of photoactive agent (69) having an inlet in openable flow communication with an inlet flow path and an outlet in openable flow communication with an outlet flow path, wherein either one of said inlet and outlet is in direct flow communication with said processing cassette (23R), and wherein said container of photoactive agent (69) is located between said processing cassette (23R) and said treatment container (68) or wherein said container of photoactive agent (69) is located between said processing cassette (23R) and said container (66) including plasma and/or saline.
h) a frangible cannula (71') for preventing flow of photoactive agent from said container of photoactive agent (69) to said treatment container (68).

2. The disposable fluid circuit of Claim 1 further comprising a frangible cannula (71', 71") for preventing flow to said container of photoactive agent (69).

3. The disposable fluid circuit of Claim 2 wherein at least one of said frangible cannulas (71', 71") is disposed in at least said inlet or outlet of the container of photoactive agent (69).

4. The disposable fluid circuit of Claim 1 comprising a frangible cannula (71', 71") located in a flow path between said container of photoactive agent (69) and said processing cassette (23R) and a frangible cannula (71', 71") located in a flow path between said container of photoactive agent (69) and said treatment container (68).

5. The disposable fluid circuit of Claim 1 comprising a frangible cannula (71', 71") located in a flow path between said container (66) including plasma and/or saline, for adjusting the volume of a collected component in said treatment container and container of photoactive agent (69) and a frangible cannula (71', 71") located in a flow path between said container of photoactive agent (69) and said treatment container (68).

6. A system for performing a therapeutic cellular treatment comprising:
a) a reusable separator unit for separating blood into blood components;
b) a reusable irradiation unit for treating a separated blood component;
c) a disposable fluid circuit (200', 200") for association with a cell separator (10) and a separate irradiation device comprising:
i. a patient access device for establishing flow communication with a patient;
ii. a cell separator (10) comprising a separation chamber (12) in openable fluid communication with said patient access device;
iii. a treatment container (68) comprising walls made of a material that is transparent to light of a selected wavelength;
iv. a processing cassette (23R) including a molded plastic body with integrally molded liquid flow channels and valve stations molded into the backside of the cassette body;
v. tubing defining a sterile flow path between said separation
chamber (12), said processing cassette (23R) and said treatment container (68), said flow path having length sufficient to allow for placement of said separation chamber within said separator and allow for placement of said treatment container in said separate irradiation device;
vi. a container of photoactive agent integral with said circuit, said container of photoactive agent having an inlet in openable flow communication with an inlet flow path and an outlet in openable flow communication with an outlet flow path, wherein either of said inlet and outlet is in direct flow communication with said processing cassette; and
vii. a frangible cannula for preventing flow of photoactive agent from said container of photoactive agent to said treatment container;
d) an automated cannula breaker configured to receive said cannula;
e) a sensor configured to sense a separated blood component exiting the separation chamber; and
f) a controller configured to effect the separation of blood into blood components, effect the conveying of the separated blood components through the disposable fluid circuit, effect the breaking of the frangible cannulas when the sensor senses the separated blood component exiting the separation chamber and, optionally, to effect the treatment of said separated components.

7. The system of Claim 6 wherein said disposable fluid circuit further comprises a frangible cannula (71', 71") for preventing flow to said container of photoactive agent (69) or preventing flow from said container of photoactive agent (69).

8. The system of Claim 7 further comprising an automated cannula breaker (250) to receive one or more cannulas.

9. The system of Claim 6 wherein said automated cannula breaker (250) is carried by said reusable separator unit.

10. The system of Claim 6 wherein said automated cannula breaker (250) is carried by said reusable irradiation unit.

11. The system of Claim 6 wherein said controller is configured to initiate breaking of one or more cannulas when a pre-determined volume of a target blood component has been collected in said treatment container (68).

## Patentansprüche

1. Einweg-Fluidkreislauf (200', 200") zur kombinierten Zuordnung zu einem Zellseparator und einer separaten Bestrahlungsvorrichtung, umfassend:
a) eine Patientenzugangsvorrichtung zum Herstellen einer Strömungsverbindung mit einem Patienten;
b) eine Zelltrennkammer (12) in öffenbarer Fluidverbindung mit der Patientenzugangsvorrichtung;
c) einen Behandlungsbehälter (68), umfassend Wände aus einem Material, das für Licht einer ausgewählten Wellenlänge transparent ist;
d) eine Verarbeitungsfluid-Strömungskassette (23R), umfassend einen geformten Kunststoffkörper mit einstückig geformten Fluidströmungskanälen und Ventilstationen, die in die Rückseite des Kassettenkörpers geformt sind;
e) ferner umfassend einen Behälter (66), der Plasma und/oder Kochsalzlösung enthält, zum Anpassen des Volumens einer gesammelten Komponente in dem Behandlungsbehälter (68) in öffenbarer Strömungsverbindung mit der Verarbeitungsfluid-Strömungskassette (23R) und dem Behandlungsbehälter (68),
f) Schläuche, die einen sterilen Strömungsweg zwischen der Trennkammer, der Verarbeitungskassette (23R) und dem Behandlungsbehälter (68) definieren, wobei der Strömungsweg eine Länge aufweist, die ausreicht, um eine Platzierung der Trennkammer innerhalb des Separators zu ermöglichen und eine Platzierung des Behandlungsbehälters in der separaten Bestrahlungsvorrichtung zu ermöglichen;
g) einen Behälter für photoaktives Mittel (69), der fest mit dem Kreislauf verbunden ist, wobei der Behälter für photoaktives Mittel (69) einen Einlass in öffenbarer Strömungsverbindung mit einem Einlassströmungsweg und einen Auslass in öffenbarer Strömungsverbindung mit einem Auslassströmungsweg aufweist, wobei entweder der Einlass oder der Auslass in direkter Strömungsverbindung mit der Verarbeitungskassette (23R) steht, und wobei der Behälter für photoaktives Mittel (69) zwischen der Verarbeitungskassette (23R) und dem Behandlungsbehälter (68) angeordnet ist oder wobei der Behälter für photoaktives Mittel (69) zwischen der Verarbeitungskassette (23R) und dem Behälter (66), der Plasma und/oder Kochsalzlösung enthält, angeordnet ist,
h) eine zerbrechliche Kanüle (71') zum Verhindern eines Flusses von photoaktivem Mittel aus dem Behälter für photoaktives Mittel (69) zu dem Behandlungsbehälter (68).

2. Einweg-Fluidkreislauf nach Anspruch 1, ferner umfassend eine zerbrechliche Kanüle (71', 71") zum Verhindern eines Flusses zu dem Behälter für photoaktives Mittel (69).

3. Einweg-Fluidkreislauf nach Anspruch 2, wobei mindestens eine der zerbrechlichen Kanülen (71', 71") zumindest in dem Einlass oder dem Auslass des Behälters für photoaktives Mittel (69) angeordnet ist.

4. Einweg-Fluidkreislauf nach Anspruch 1, umfassend eine zerbrechliche Kanüle (71', 71"), die in einem Strömungsweg zwischen dem Behälter für photoaktives Mittel (69) und der Verarbeitungskassette (23R) angeordnet ist, und eine zerbrechliche Kanüle (71', 71"), die in einem Strömungsweg zwischen dem Behälter für photoaktives Mittel (69) und dem Behandlungsbehälter (68) angeordnet ist.

5. Einweg-Fluidkreislauf nach Anspruch 1, umfassend eine zerbrechliche Kanüle (71', 71"), die in einem Strömungsweg zwischen dem Behälter (66), der Plasma und/oder Kochsalzlösung enthält, zum Anpassen des Volumens einer gesammelten Komponente in dem Behandlungsbehälter und dem Behälter für photoaktives Mittel (69) angeordnet ist, und eine zerbrechliche Kanüle (71', 71"), die in einem Strömungsweg zwischen dem Behälter für photoaktives Mittel (69) und dem Behandlungsbehälter (68) angeordnet ist.

6. System zum Durchführen einer therapeutischen Zellbehandlung, umfassend:
a) eine wiederverwendbare Separatoreinheit zum Trennen von Blut in Blutkomponenten;
b) eine wiederverwendbare Bestrahlungseinheit zum Behandeln einer abgetrennten Blutkomponente;
c) einen Einweg-Fluidkreislauf (200', 200") zur Zuordnung zu einem Zellseparator (10) und einer separaten Bestrahlungsvorrichtung, umfassend:
i) eine Patientenzugangsvorrichtung zum Herstellen einer Strömungsverbindung mit einem Patienten;
ii) einen Zellseparator (10), umfassend eine Trennkammer (12) in öffenbarer Fluidverbindung mit der Patientenzugangsvorrichtung;
iii) einen Behandlungsbehälter (68), umfassend Wände aus einem Material, das für Licht einer ausgewählten Wellenlänge transparent ist;
iv) eine Verarbeitungskassette (23R), umfassend einen geformten Kunststoffkörper mit einstückig geformten Fluidströmungskanälen und Ventilstationen, die in die Rückseite des Kassettenkörpers geformt sind;
v) Schläuche, die einen sterilen Strömungsweg zwischen der Trennkammer (12), der Verarbeitungskassette (23R) und dem Behandlungsbehälter (68) definieren, wobei der Strömungsweg eine Länge aufweist, die ausreicht, um eine Platzierung der Trennkammer innerhalb des Separators zu ermöglichen und eine Platzierung des Behandlungsbehälters in der separaten Bestrahlungsvorrichtung zu ermöglichen;
vi) einen Behälter für photoaktives Mittel, der mit dem Kreislauf fest verbunden ist, wobei der Behälter für photoaktives Mittel einen Einlass in öffenbarer Strömungsverbindung mit einem Einlassströmungsweg und einen Auslass in öffenbarer Strömungsverbindung mit einem Auslassströmungsweg aufweist, wobei entweder der Einlass oder der Auslass in direkter Strömungsverbindung mit der Verarbeitungskassette steht; und
vii) eine zerbrechliche Kanüle zum Verhindern eines Flusses von photoaktivem Mittel aus dem Behälter für photoaktives Mittel zu dem Behandlungsbehälter;
d) einen automatisierten Kanülenbrecher, der dazu konfiguriert ist, die Kanüle aufzunehmen;
e) einen Sensor, der dazu konfiguriert ist, eine abgetrennte Blutkomponente zu erfassen, die aus der Trennkammer austritt; und
f) eine Steuerung, die dazu konfiguriert ist, die Trennung von Blut in Blutkomponenten zu bewirken, das Befördern der abgetrennten Blutkomponenten durch den Einweg-Fluidkreislauf zu bewirken, das Brechen der zerbrechlichen Kanülen zu bewirken, wenn der Sensor die abgetrennte Blutkomponente erfasst, die aus der Trennkammer austritt, und optional die Behandlung der abgetrennten Komponenten zu bewirken.

7. System nach Anspruch 6, wobei der Einweg-Fluidkreislauf ferner eine zerbrechliche Kanüle (71', 71") zum Verhindern eines Flusses zu dem Behälter für photoaktives Mittel (69) oder zum Verhindern eines Flusses aus dem Behälter für photoaktives Mittel (69) umfasst.

8. System nach Anspruch 7, ferner umfassend einen automatisierten Kanülenbrecher (250) zum Aufnehmen einer oder mehrerer Kanülen.

9. System nach Anspruch 6, wobei der automatisierte Kanülenbrecher (250) von der wiederverwendbaren Separatoreinheit getragen wird.

10. System nach Anspruch 6, wobei der automatisierte Kanülenbrecher (250) von der wiederverwendbaren Bestrahlungseinheit getragen wird.

11. System nach Anspruch 6, wobei die Steuerung dazu konfiguriert ist, das Brechen einer oder mehrerer Kanülen einzuleiten, wenn ein vorbestimmtes Volumen einer Ziel-Blutkomponente in dem Behandlungsbehälter (68) gesammelt worden ist.

## Revendications

1. Circuit fluidique jetable (200', 200") destiné à une association combinée avec un séparateur de cellules et un dispositif d'irradiation séparé, comprenant :
a) un dispositif d'accès au patient destiné à établir une communication d'écoulement avec un patient ;
b) une chambre de séparation cellulaire (12) en communication fluidique ouvrable avec ledit dispositif d'accès au patient ;
c) un récipient de traitement (68) comprenant des parois constituées d'un matériau transparent à de la lumière d'une longueur d'onde choisie ;
d) une cassette d'écoulement de fluide de traitement (23R) comprenant un corps en plastique moulé avec des canaux d'écoulement de liquide moulés d'un seul tenant et des postes de vanne moulés dans la face arrière du corps de cassette ;
e) comprenant en outre un récipient (66) contenant du plasma et/ou de la solution saline, destiné à ajuster le volume d'un composant recueilli dans ledit récipient de traitement (68), en communication d'écoulement ouvrable avec ladite cassette d'écoulement de fluide de traitement (23R) et ledit récipient de traitement (68),
f) une tubulure définissant un trajet d'écoulement stérile entre ladite chambre de séparation, ladite cassette de traitement (23R) et ledit récipient de traitement (68), ledit trajet d'écoulement ayant une longueur suffisante pour permettre le placement de ladite chambre de séparation à l'intérieur dudit séparateur et permettre le placement dudit récipient de traitement dans ledit dispositif d'irradiation séparé ;
g) un récipient d'agent photoactif (69) solidaire dudit circuit, ledit récipient d'agent photoactif (69) ayant une entrée en communication d'écoulement ouvrable avec un trajet d'écoulement d'entrée et une sortie en communication d'écoulement ouvrable avec un trajet d'écoulement de sortie, dans lequel l'une quelconque desdites entrée et sortie est en communication d'écoulement directe avec ladite cassette de traitement (23R), et dans lequel ledit récipient d'agent photoactif (69) est situé entre ladite cassette de traitement (23R) et ledit récipient de traitement (68), ou dans lequel ledit récipient d'agent photoactif (69) est situé entre ladite cassette de traitement (23R) et ledit récipient (66) contenant du plasma et/ou de la solution saline,
h) une canule frangible (71') destinée à empêcher de l'écoulement d'agent photoactif depuis ledit récipient d'agent photoactif (69) vers ledit récipient de traitement (68).

2. Circuit fluidique jetable selon la revendication 1, comprenant en outre une canule frangible (71', 71") destinée à empêcher de l'écoulement vers ledit récipient d'agent photoactif (69).

3. Circuit fluidique jetable selon la revendication 2, dans lequel au moins l'une desdites canules frangibles (71', 71") est disposée dans au moins ladite entrée ou sortie du récipient d'agent photoactif (69).

4. Circuit fluidique jetable selon la revendication 1, comprenant une canule frangible (71', 71") située dans un trajet d'écoulement entre ledit récipient d'agent photoactif (69) et ladite cassette de traitement (23R), et une canule frangible (71', 71") située dans un trajet d'écoulement entre ledit récipient d'agent photoactif (69) et ledit récipient de traitement (68).

5. Circuit fluidique jetable selon la revendication 1, comprenant une canule frangible (71', 71") située dans un trajet d'écoulement entre ledit récipient (66) contenant du plasma et/ou de la solution saline, destiné à ajuster le volume d'un composant recueilli dans ledit récipient de traitement, et le récipient d'agent photoactif (69), et une canule frangible (71', 71") située dans un trajet d'écoulement entre ledit récipient d'agent photoactif (69) et ledit récipient de traitement (68).

6. Système destiné à réaliser un traitement cellulaire thérapeutique, comprenant :
a) une unité de séparateur réutilisable destinée à séparer du sang en des composants sanguins ;
b) une unité d'irradiation réutilisable destinée à traiter un composant sanguin séparé ;
c) un circuit fluidique jetable (200', 200") destiné à être associé à un séparateur de cellules (10) et à un dispositif d'irradiation séparé, comprenant :
i) un dispositif d'accès au patient destiné à établir une communication d'écoulement avec un patient ;
ii) un séparateur de cellules (10) comprenant une chambre de séparation (12) en communication fluidique ouvrable avec ledit dispositif d'accès au patient ;
iii) un récipient de traitement (68) comprenant des parois constituées d'un matériau transparent à de la lumière d'une longueur d'onde choisie ;
iv) une cassette de traitement (23R) comprenant un corps en plastique moulé avec des canaux d'écoulement de liquide moulés d'un seul tenant et des postes de vanne moulés dans la face arrière du corps de cassette ;
v) une tubulure définissant un trajet d'écoulement stérile entre ladite chambre de séparation (12), ladite cassette de traitement (23R) et ledit récipient de traitement (68), ledit trajet d'écoulement ayant une longueur suffisante pour permettre le placement de ladite chambre de séparation à l'intérieur dudit séparateur et permettre le placement dudit récipient de traitement dans ledit dispositif d'irradiation séparé ;
vi) un récipient d'agent photoactif solidaire dudit circuit, ledit récipient d'agent photoactif ayant une entrée en communication d'écoulement ouvrable avec un trajet d'écoulement d'entrée et une sortie en communication d'écoulement ouvrable avec un trajet d'écoulement de sortie, dans lequel l'une quelconque desdites entrée et sortie est en communication d'écoulement directe avec ladite cassette de traitement ; et
vii) une canule frangible destinée à empêcher de l'écoulement d'agent photoactif depuis ledit récipient d'agent photoactif vers ledit récipient de traitement ;
d) un dispositif automatisé de rupture de canule configuré pour recevoir ladite canule ;
e) un capteur configuré pour détecter un composant sanguin séparé sortant de la chambre de séparation ; et
f) un contrôleur configuré pour effectuer la séparation du sang en des composants sanguins, effectuer l'acheminement des composants sanguins séparés à travers le circuit fluidique jetable, effectuer la rupture des canules frangibles lorsque le capteur détecte le composant sanguin séparé sortant de la chambre de séparation et, facultativement, pour effectuer le traitement desdits composants séparés.

7. Système selon la revendication 6, dans lequel ledit circuit fluidique jetable comprend en outre une canule frangible (71', 71") destinée à empêcher de l'écoulement vers ledit récipient d'agent photoactif (69) ou à empêcher de l'écoulement depuis ledit récipient d'agent photoactif (69).

8. Système selon la revendication 7, comprenant en outre un dispositif automatisé de rupture de canule (250) destiné à recevoir une ou plusieurs canules.

9. Système selon la revendication 6, dans lequel ledit dispositif automatisé de rupture de canule (250) est porté par ladite unité de séparateur réutilisable.

10. Système selon la revendication 6, dans lequel ledit dispositif automatisé de rupture de canule (250) est porté par ladite unité d'irradiation réutilisable.

11. Système selon la revendication 6, dans lequel ledit contrôleur est configuré pour initier la rupture d'une ou plusieurs canules lorsqu'un volume prédéterminé d'un composant sanguin cible a été recueilli dans ledit récipient de traitement (68).
